Europäisches Patentamt

European Patent Office

Office européen des brevets

(11) Veröffentlichungsnummer: **0 419 770 A1**

## (12) EUROPÄISCHE PATENTANMELDUNG

(21) Anmeldenummer: 90111965.1

(22) Anmeldetag: 23.06.90

(51) Int. Cl.⁵: **B65D 85/16**

(30) Priorität: 27.09.89 DE 8911486 U

(43) Veröffentlichungstag der Anmeldung:
03.04.91 Patentblatt 91/14

(84) Benannte Vertragsstaaten:
AT BE CH DE DK ES FR GB GR IT LI NL SE

(71) Anmelder: Christian Senning
Verpackungsautomaten GmbH & Co.
Kalmsweg 1o
W-2800 Bremen 21(DE)

(72) Erfinder: Lippert, Wolfgang
Reddersenstr. 31
2800 Bremen(DE)
Erfinder: Hadzelek, Franz
Oslebshauser Heerstr. 71 C
2800 Bremen 21(DE)

(74) Vertreter: Eisenführ, Speiser & Strasse
Martinistrasse 24
W-2800 Bremen 1(DE)

(54) Weichpackung nicht-rechteckiger Flachgegenstände.

(57) Zur Herstellung einer Folien-Weichpackung für mehrere nicht-rechteckige, flexible Flachgegenstände, insbesondere für längliche und endseits gerundete Slip-Einlagen (2) werden die Flachgegenstände zur Rechteckform gefaltet und von einer quaderförmigen, am Stapel der übereinanderliegend gefalteten Flachgegenstände relativ eng anliegenden Verpackungshüllen (20) umgeben, wobei die Verpackungshülle über die gesamte Breite einer Seitenfläche und wenigstens einem Teil ihrer Tiefe zu öffnen und die Öffnung wiederverschließbar ausgebildet ist. Insbesondere erfolgt die Faltung derart, daß die Endbereiche der Flachgegenstände gegensinnig gefaltet und nebeneinander auf dem Hauptbereich liegen.

Fig. 3

Fig. 4

## WEICHPACKUNG NICHT-RECHTECKIGER FLACHGEGENSTÄNDE

Die Erfindung betrifft eine Weichpackung nicht-rechteckiger, flexibler Flachgegenstände, insbesondere länglicher, endseits gerundeter Slip-Einlagen, die in gefaltetem Zustand mit Folienmaterial umhüllt sind.

Bei den hier insbesondere angesprochenen Slip-Einlagen handelt es sich um flache, mehrlagige Zuschnitte aus saugfähigem Tissue-Material von länglicher Form. Sie sind an ihren kurzen Enden aus Gründen der Anpassung an den Körper und die Kleidung der Benutzerin stark - bis nahe an die Halbkreisform - gerundet. Zum Vertrieb werden im allgemeinen Stapel dieser Einlagen in Kartons verpackt und verkauft.

Wegen der Unhandlichkeit solcher Vorratspackungen sind daneben Kleinpackungen mit einzelnen Slip-Einlagen entwickelt worden. Dabei ist die Längserstreckung der Einlage durch Querfalten auf etwa 1/3 ihrer Länge verringert und die dreischichtig zusammengefaltete Einlage in einer beutelartigen Folienpackung untergebracht.

Es besteht jedoch auch ein Bedarf an einer handhabbaren und vertriebsfähigen Einheit mehrerer Slip-Einlagen, weil die Einzelverpackung kostenaufwendig und die Mitnahme mehrerer einzeln verpackter Slip-Einlagen umständlich ist. Letzteres beansprucht außerdem unverhältnismäßig viel Raum. Schließlich liegen bei der bekannten Querfaltung die Falze an solchen Stellen der Slip-Einlagen, wo sie beim Tragen störend in Erscheinung treten.

Das Verpacken eines Stapels von Slip-Einlagen nach Art gefalteter Papiertaschentücher, welches sich im Hinblick auf Ähnlichkeiten in Gestalt und Konsistenz anzubieten scheint, ist tatsächlich nicht möglich, weil sich die bei Taschentuchpäcken heute allgemein übliche Weichverpackung um die nicht-rechteckigen Slip-Einlagen nicht - jedenfalls nicht zuverlässig -schließen läßt. Schon gar nicht läßt sich der weitergehende Wunsch erfüllen, die Weichverpackungshülle eines kleinen Stapels von Slip-Einlagen so auszuführen, daß sie nach erstmaliger Entnahme einer Einlage wieder verschlossen und dieser Vorgang bis zum völligen Aufbrauch mehrfach wiederholt werden kann.

Aus dieser Schwierigkeit weist die Erfindung einen Weg. Sie besteht darin, daß eine Mehrzahl zur Rechteckform gefalteter Flachgegenstände übereinander gestapelt und von einer quaderförmigen, am Stapel relativ eng anliegenden Verpackungshülle umgeben ist, und daß die Verpackungshülle zum Öffnen über die gesamte Breite einer Quader-Seitenfläche und wenigstens einen Teil ihrer Tiefe sowie wiederverschließbar ausgebildet ist.

Die Erfindung macht sich dabei die - an sich bekannte - Faltung der Slip-Einlage in neuartiger Weise zunutze: Die Rechteckform, die sich beim Falten der Flachgegenstände - namentlich also von Slip-Einlagen - ergibt, erlaubt die Bildung eines quaderförmigen Stapels und dessen Einschließen in eine allseits eng anliegende, wiederverschließbare Weichpackung. Zu beachten ist allerdings, daß eine Entnahme der gefalteten Gegenstände wegen der im Knickbereich eintretenden Versteifung nur möglich ist, wenn die Zugang gebende Seitenwand über die gesamte Breite (des Gegenstandes und damit der Packung) geöffnet wird, um ein problemloses und die Verpackung und/oder den Gegenstand nicht beschädigendes Entnehmen zu gewährleisten.

Das Falten etwa von Slip-Einlagen kann in der bekannten Weise derart erfolgen, daß die gegensinnig gefalteten Endbereiche übereinanderliegen. Eine - bei gleicher Anzahl - flachere, aber natürlich längere Packung ergibt sich, wenn die umgefalteten Endbereiche nebeneinanderliegen. Dadurch gelangen außerdem die Querfalze an Stellen in Längsrichtung der Einlagen, wo sie beim Tragen nicht stören.

Im letzteren Falle könnte man auch solche Slip-Einlagen (oder vergleichbare Gegenstände) vorteilhaft verpacken, die im Mittelbereich verdickt sind: Dann werden nur so kleine Endbereiche umgefaltet, daß die Rechteckform erreicht wird und die umgefalteten Abschnitte neben dem verdickten Mittelabschnitt liegen und sich eine im wesentlichen gleichförmige Dicke ergibt, was auch insoweit zur Quaderform des Verpackungsstapels führt.

Die Erfindung wird nachfolgend anhand der in den Zeichnungen dargestellten Ausführungsbeispiele näher erläutert. Es zeigen:

Fig. 1 eine Slip-Einlage im ungefalteten Zustand;

Fig. 2 eine Slip-Einlage im gefalteten Zustand;

Fig. 3 einen Stapel von zusammengefalteten Slip-Einlagen;

Fig. 4 eine Weichpackung zur Aufnahme des in Figur 3 dargestellten Stapels von gefalteten Slip-Einlagen in perspektivischer Darstellung;

Fig. 5 einen ausgebreiteten Zuschnitt aus einer Kunststoffolie zur Herstellung der in Figur 4 dargestellten Weichpackung; und

Fig. 6 einen ausgebreiteten Zuschnitt aus einer Kunststoffolie zur Herstellung einer Weichpackung in einer weiteren Ausführung.

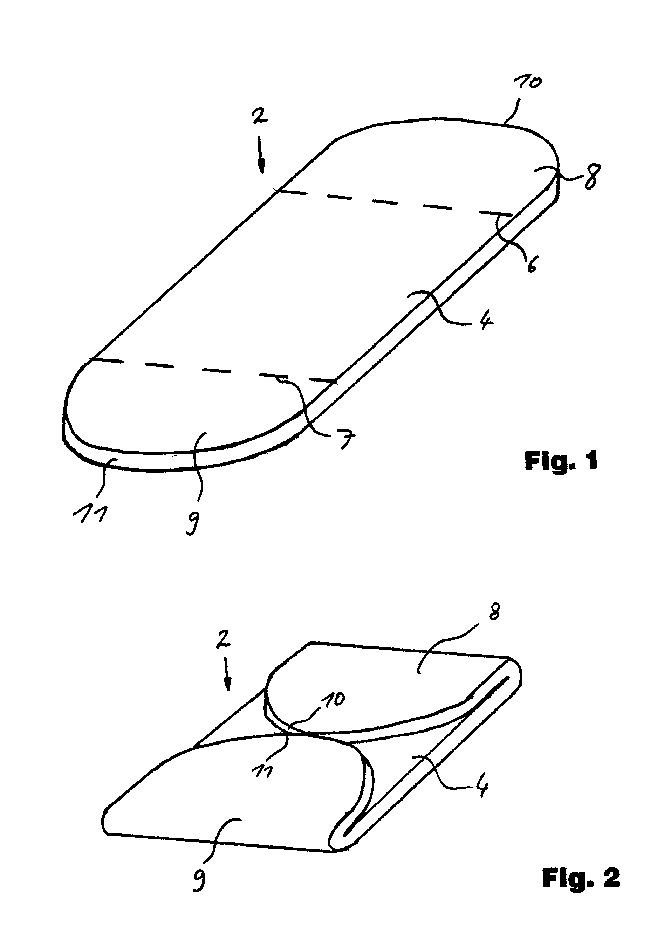

In Figur 1 ist perspektivisch eine Slip-Einlage 2 dargestellt, die einen Hauptbereich 4 und zwei gegenüberliegende und über Knicklinien 6, 7 mit dem Hauptbereich 4 verbundene Endbereiche 8, 9 aufweist. Während der Hauptbereich 4 eine im wesentlichen rechteckige Form besitzt, sind die sich daran anschließenden Endbereiche 8, 9 an ihren freien Seitenkanten 10, 11 aus Gründen der Anpassung stark abgerundet. Bei dem in Figur 1 dargestellten Ausführungsbeispiel sind die bei den Endbereiche 8, 9 im wesentlichen halbkreisförmig ausgebildet.

Wie Figur 1 zeigt, besitzt die gesamte Slip-Einlage 2 eine flache, längliche Form. Sie besteht aus einem flachen, mehrlagigen Zuschnitt aus saugfähigem Tissue-Material. Die Dicke der in Figur 1 dargestellten Slip-Einlage 2 ist über ihre gesamte Länge, also sowohl in ihrem Hauptbereich 4 als auch in ihren Endbereichen 8, 9 gleich groß. Es ist aber auch denkbar, den Hauptbereich 4 dicker und somit saugfähiger als die beiden Endbereiche 8, 9 auszubilden.

Zur Anordnung in einer noch zu beschreibenden Weichpackung wird die Slip-Einlage 2 gemäß Figur 2 gefaltet. Dabei werden die beiden Endbereiche 8, 9 um die Knicklinien 6, 7, die im wesentlichen parallel zueinander und rechtwinklig zur Längserstreckung der Slip-Einlage 2 verlaufen, auf den Hauptbereich 4 gegensinnig um 180° zurückgefaltet. Bei dem in Figur 2 dargestellten gefalteten Zustand liegen die beiden Endbereiche 8, 9 nebeneinander auf dem Hauptbereich 4, wobei die freien Seitenkanten 10, 11 der Endbereiche 8, 9 aufeinander zu und benachbart zueinander angeordnet sind. Eine derartige Faltung ist insbesondere dann vorteilhaft, wenn der Mittelbereich 4 gegenüber den Endbereichen 8, 9 verdickt ist (in den Figuren nicht dargestellt). Da dann die umgefalteten Endbereiche neben dem verdickten Mittelbereich liegen, ergibt sich eine im wesentlichen gleichförmige Dicke der zusammengefalteten Slip-Einlage. Demgegenüber ist es auch möglich, die Slip-Einlage so zu falten, daß die gegensinnig gefalteten Endbereiche übereinanderliegen. Diese Faltung hat den Vorteil, daß die Slip-Einlage im gefalteten Zustand nur eine besonders geringe Länge aufweist. Auf jeden Fall muß aber die endseitig abgerundete Slip-Einlage 2 so gefaltet werden, daß sie im gefalteten Zustand eine Rechteckform erhält, wie es beispielhaft in Figur 2 gezeigt ist.

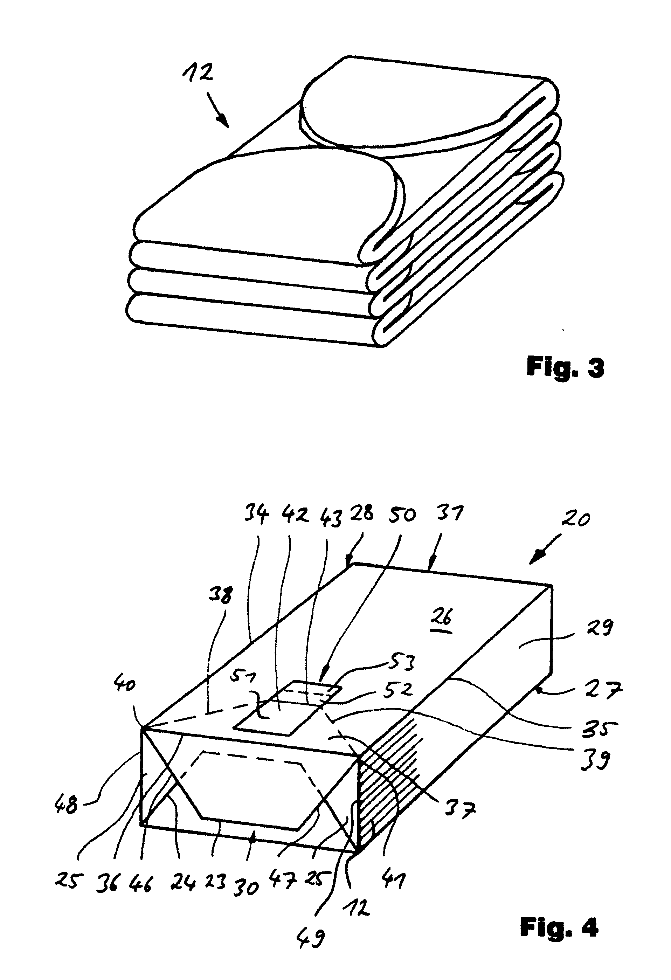

Die Rechteckform, die sich beim Falten der Slip-Einlagen 2 in der zuvor beschriebenen Weise ergibt, erlaubt die Bildung eines quaderförmigen Stapels 12 aus mehreren übereinanderliegenden, gefalteten Slip-Einlagen, wie in Figur 3 dargestellt ist.

Die gemäß Figur 2 zur Rechteckform gefalteten und gemäß Figur 3 übereinander gestapelten Slip-Einlagen 2 sind im verpackten Zustand von einer quaderförmigen und am Stapel 12 relativ eng anliegenden Weichpackung 20 umgeben, die in Figur 4 in Außenansicht dargestellt ist. Der in der Weichpackung 20 enthaltene Stapel 12 aus den Slip-Einlagen ist in Figur 4 nur angedeutet.

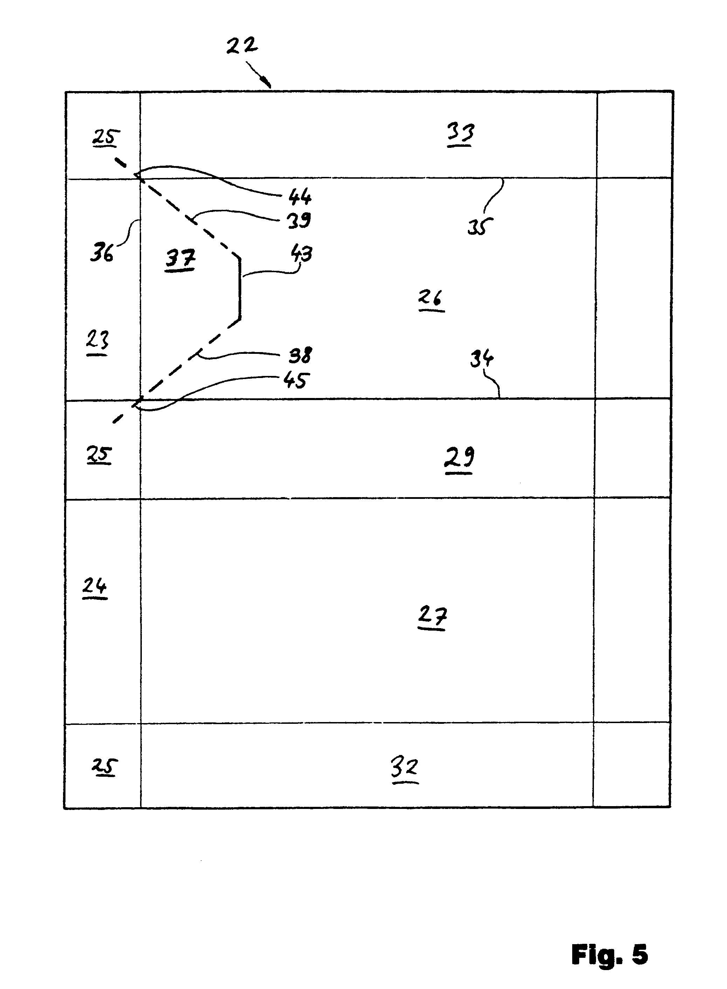

Die Weichpackung 20 besteht aus einer Verpackungsfolie, die aus einem einstückigen, rechteckigen Zuschnitt 22 aus einer schweißbaren oder klebbaren Kunststoffolie, zum Beispiel Polyethylen, gebildet ist. Der Zuschnitt 22 ist in Figur 5 in Draufsicht dargestellt, bevor er zur Weichpackung zusammengefaltet und -geklebt wird. Der Zuschnitt 22 wird zunächst schlauchförmig um den Stapel 12 aus Slip-Einlagen herumgelegt. An den Enden überstehende Stirnlappen 23, 24 und 25 werden sodann gegen den Stapel 12 umgefaltet. Die einzelnen durch entsprechende Linien in Figur 5 markierten Felder des Zuschnitts 22 bilden dadurch eine Vorderwand 26, eine Rückwand 27, demgegenüber schmalere Seitenwände 28 und 29 sowie Stirnwände 30 und 31.

Die Stirnwände 30, 31 werden durch die entsprechend gefalteten Stirnlappen 23, 24 und 25 gebildet, von denen der der Vorderwand 26 zugeordnete (äußere) Stirnlappen 23 ebenso wie der entsprechende, an die Rückwand 27 angesetzte Stirnlappen 24 eine trapezförmige Gestalt durch den Faltvorgang erhalten. Die Stirnlappen 23, 24 und 25 sind durch thermische Schweißung oder ggf. auch durch Klebung miteinander verbunden.

Seitenstreifen 32 und 33 des Zuschnitts 22 bilden im Bereich der Seitenwand 28 eine hier im einzelnen nicht gezeigte Überlappung. Die Seitenstreifen 32, 33 sind dabei ebenfalls durch thermische Schweißung oder ggf. durch Klebung miteinander verbunden.

Die Vorderwand 26 ist durch Längskanten 34 und 35 gegenüber den Seitenwänden 28 und 29 und durch eine Querkante 36 gegenüber der Stirnwand 30 abgegrenzt.

Bei dem hier gezeigten Ausführungsbeispiel ist die rechteckige Vorderwand 36 im der Stirnwand 30 zugekehrten Bereich mit einer sich über die gesamte Breite der Vorderwand 26 erstreckenden Aufreißlasche 37 versehen. Diese ist demnach so breit wie die Vorderwand 26. Die Aufreißlasche 37 ist durch Reißlinien begrenzt, die aufgrund einer Materialschwächung der Kunststoffolie den Aufreißvorgang ermöglichen. Bei dem dargestellten Ausführungsbeispiel wird die Aufreißlasche 37 durch Perforationslinien 38, 39 begrenzt, die in Aufreißrichtung, also in Richtung zur Stirnwand 30 verlaufen.

Bei dem in den Figuren 4 und 5 gezeigten Ausführungsbeispiel erhält die Aufreißlasche 37 eine im wesentlichen trapezförmige Gestalt, da sie durch zwei jeweils zu den Ecken 40, 41 der Vorderwand 26 laufende Perforationslinien 38, 39 gebildet wird. Ein etwa mittig zur Vorderwand 26 liegendes Laschenende 42 wird hier durch einen die zugekehrten Enden der Perforationslinien 38, 39 miteinander verbindenden, ununterbrochenen Trennschnitt 43 markiert. Dieser erleichtert den kritischen Anfang des Aufreißvorganges durch Erfassen und Hochziehen des Laschenendes 42.

Um eine die Entnahme des Packungsinhaltes erleichternde Öffnung durch die Aufreißlasche 37 zu schaffen, werden die Perforationslinien 38, 39 durch Anschlußperforationen 44, 45 im Bereich der Stirnwand 30 fortgesetzt. Bei dem in Figur 5 dargestellten Ausführungsbeispiel schließen sich die Anschlußperforationen 44, 45 geradlinig an die Perforationslinien 38, 39 an, wobei sie mit den seitlichen Faltkanten 46, 47 (vgl. Figur 4) des äußeren Stirnlappens 23 zusammenfallen.

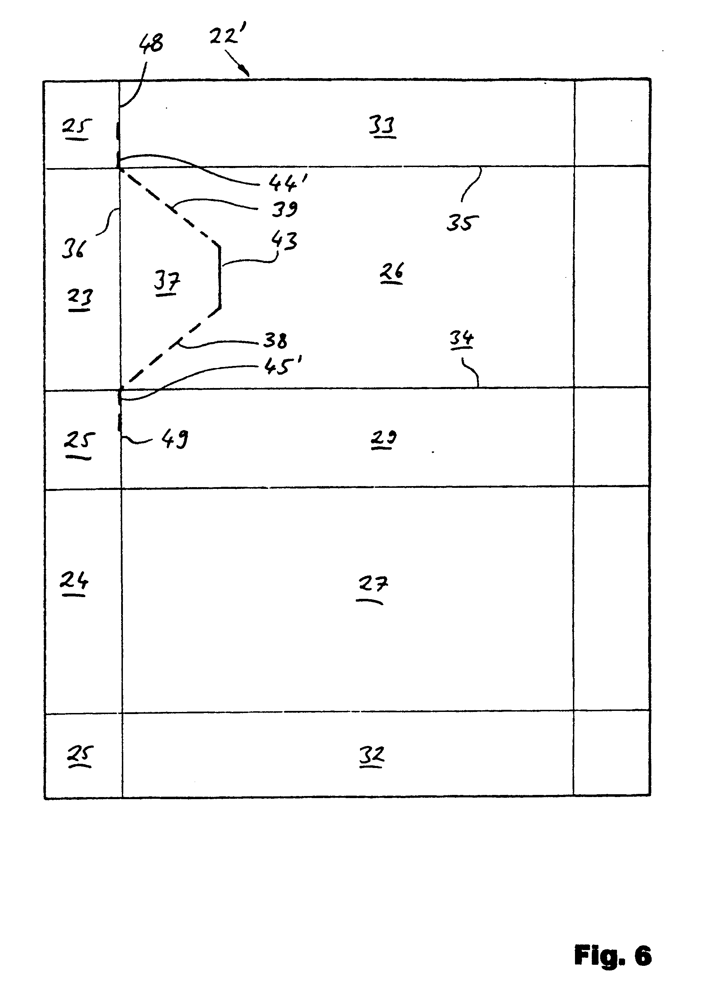

Figur 6 zeigt ein weiteres Ausführungsbeispiel eines Zuschnittes 22', welcher sich von dem in Figur 5 dargestellten Zuschnitt nur dadurch unterscheidet, daß die Anschlußperforationen 44', 45' entlang der die Seitenwände 28, 29 mit der Stirnwand 30 verbindenden Querkanten 48, 49 verlaufen.

Auf jeden Fall ist es wichtig, daß die sich an die Perforationslinien 38, 39 anschließenden Anschlußperforationen so geführt sind, daß beim Öffnen der Aufreißlasche 37 auch der benachbarte Abschnitt der Stirnwand 30 über die gesamte Breite mit geöffnet wird, damit die Slip-Einlagen leicht zu entnehmen sind.

Es ist auch denkbar, die Anschlußperforationen 44, 45 bzw. 44', 45' auch als durchgehende Trennschnitte auszuführen.

Wie bereits erwähnt, sind die Perforationslinien 38, 39 und einschließlich des Trennschnittes 43 so angeordnet, daß die Aufreißlasche 37 eine Trapezform erhält. Es sind jedoch auch andere geometrische Formen denkbar, wie z. B. Bogenformen mit nach außen und/oder nach innen gerichteten konkaven Abschnitten. Es muß lediglich bei der Ausbildung der Aufreißlasche 37 darauf geachtet werden, daß sie über die gesamte Breite einer Quader-Seitenfläche wie z. B. bis zu den Längskanten 34, 35 der Vorderwand 26 bei der in Figur 4 dargestellten Weichpackung 20 und über wenigstens einen Teil der sich daran anschließenden Querwand wie z. B. der Stirnwand 30 ausgebildet ist.

Damit die Aufreißlasche 37 nach dem Aufreißen wieder verschließbar ist, besitzt sie ein Verschlußorgan in Form eines Klebestreifens 50, wie Figur 4 zeigt. Dieser Klebestreifen 50 ist mit einem Haftabschnitt 51 durch Klebung mit der Aufreißlasche 37 verbunden. Der daran anschließende Abziehteil 52 ist mit erheblich kleinerer Haftfläche ausgebildet und haftet durch Klebung lösbar am darunterliegenden Teil der Vorderwand 26. Eine am Ende des Klebestreifens 50 gebildete nichthaftende Grifflasche 53 ermöglicht das Erfassen und Abziehen des Klebestreifens 50.

Beim erstmaligen Öffnen der Weichpackung 20 wird zunächst der Abziehteil 52 von der Vorderwand 26 gelöst. Bei weiterer Abziehbewegung des Klebestreifens 50 wird die Aufreißlasche 37 entlang den Perforationslinien 38, 39 von der Vorderwand 26 gelöst, da der Klebestreifen 50 in Folge entsprechender Haftung mit der Aufreißlasche 37 in Verbindung bleibt. Außerdem wird noch der sich an die Aufreißlasche 37 anschließende Abschnitt der Stirnwand 30 entlang der Anschlußperforationen 44, 45 (vgl. Figur 5) bzw. 44', 45' (vgl. Figur 6) gelöst.

Durch diesen Aufreißvorgang wird eine von den Perforationslinien 38, 39 und Anschlußperforationen 44, 45 bzw. 44', 45' begrenzte Öffnung freigelegt, die die Entnahme der verpackten Slip-Einlagen ermöglicht. Nach einer Teilentnahme kann die Weichpackung 20 wieder verschlossen werden, und zwar mit Hilfe des Klebestreifens 50. Ein mehrfacher Öffnungs- und Schließvorgang ist somit durchführbar.

## Ansprüche

1. Weichpackung nicht-rechteckiger, flexibler Flachgegenstände, insbesondere länglicher, endseits gerundeter Slip-Einlagen (2), die in gefaltetem Zustand mit Folienmaterial (22) umhüllt sind, dadurch gekennzeichnet, daß eine Mehrzahl zur Rechteckform gefalteter Flachgegenstände (2) übereinander gestapelt und von einer quaderförmigen, am Stapel (12) relativ eng anliegenden Verpackungshülle (20) umgeben sind, und daß die Verpackungshülle (20) zum Öffnen über die gesamte Breite einer Quader-Seitenfläche (z. B. 26) und wenigstens einen Teil ihrer Tiefe (z. B. 30) sowie wiederverschließbar ausgebildet ist.

2. Weichpackung nach Anspruch 1, dadurch gekennzeichnet, daß die Flachgegenstände (2) entlang zweier paralleler Knicklinien (6, 7) gegensinnig gefaltet sind.

3. Weichpackung nach Anspruch 2, dadurch gekennzeichnet, daß die Endbereiche (8, 9) der Flachgegenstände (2) nebeneinander auf deren Hauptbereich (4) angeordnet sind.

4. Weichpackung nach Anspruch 3, dadurch gekennzeichnet, daß nur so kleine Endbereiche (8, 9) umgefaltet sind, wie zur Erreichung der Rechteckform erforderlich sind.

5. Weichpackung nach Anspruch 2, dadurch gekennzeichnet, daß die Endbereiche (8, 9) übereinander auf dem Hauptbereich (4) angeordnet sind.

**Fig. 1**

**Fig. 2**

12

**Fig. 3**

34  42  43  28  50  37                    20

38

40

26                    29

51        53
         52                    27

                                          35
                                          39

48

                                          37

25  36  46  24  23  30  47  25      41

                    12

49                                  **Fig. 4**

**Fig. 5**

**Fig. 6**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | FR-A-1 271 368 (KIMBERLY-CLARK) * Seite 1, rechte Spalte, Absätze 5,6 – Seite 2, Absatz 2; Figuren 1-4 * | 1 | B 65 D 85/16 |
| A | --- | 2,5 | |
| Y | DE-U-8 319 608 (SCHICKEDANZ) * Seite 8, Absatz 2; Figuren 1,2 * ----- | 1 | |

RECHERCHIERTE
SACHGEBIETE (Int. Cl.5)

B 65 D

Der vorliegende Recherchenbericht wurde für alle Patentansprüche erstellt

| Recherchenort | Abschlußdatum der Recherche | Prüfer |
|---|---|---|
| BERLIN | 02-11-1990 | LORENZ P A |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes
Dokument

EPO FORM 1503 03.82 (P0403)